# EUROPEAN PATENT SPECIFICATION

(11) **EP 2 849 733 B1**
(45) Date of publication and mention of the grant of the patent: **08.04.2020**
(21) Application number: 12840880.4
(22) Date of filing: 19.12.2012
(51) Int. Cl.: A61K 9/46, A61K 31/197, A61K 31/714, A61K 45/06, A61P 25/00, A61P 25/08

(54) **EFFERVESCENT PHARMACEUTICAL FORMULATIONS COMPRISING PREGABALIN AND VITAMIN B12**
BRAUSEFORMULIERUNG ENTHALTEND PREGABALIN UND VITAMIN B12
FORMULATION EFFERVESCENTE COMPRENANT DE LA PRÉGABALINE ET LA VITAMINE B12

(30) Priority: 19.12.2011 TR 201112579; 19.03.2012 TR 201203081
(43) Date of publication of application: 25.03.2015
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2012/000222
(87) International publication number: WO 2013/109199

(56) References cited:
- WO-A1-2010/150221
- WO-A2-2007/048223
- WO-A2-2011/053265
- WO-A2-2012/016683
- US-A1- 2009 304 602
- US-A1- 2010 190 752

## Description

The present invention relates to effervescent pharmaceutical formulations comprising a GABA analogue pregabalin and vitamin B₁₂ in combined form and use of these formulations in the treatment and/or prevention of epilepsy, central nervous system disorders, Parkinson's disease; Huntington's disease; tardive dyskinesia; spasticity; cerebral ischemia; postherpetic neuralgia; social phobia; fibromyalgia and spinal cord injury induced chronic pains; neuropathic pains associated with diabetic peripheral neuropathy and common anxiety disorder and anemia diseases such as macrocytic anemia, megaloblastic anemia and pernicious anemia.

Pregabalin (*Lyrica*®)*,* is a gamma-aminobutyric acid (GABA) analogue; and it has an anxiolytic, analgesic and anti-epileptic activity. Chemical structure of pregabalin having the chemical name of (S)-3-(aminomethyl)-5-methylhexanoic acid is illustrated with Formula I.

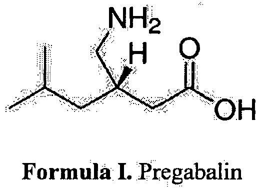

It is known that pregabalin displaces [3H]-gabapentin by binding to sub-unit of voltage-gated calcium channels in central nervous system. Pregabalin reduces release of many neurotransmitters including glutamate, noradrenaline and substance P. It is used in the treatment of epilepsy, simple or complex partial convulsion either accompanied or not by secondary generalized convulsions and neuropathic pain.

Pregabalin was first disclosed in the patent numbered EP 0641330 B1. Processes for preparation of pregabalin, pregabalin salts and use of pregabalin in the treatment of central nervous system disorders, epilepsy, Parkinson's disease, Huntington's disease, tardive dyskinesia, spasticity and cerebral ischemia are described in said patent.

In the prior art, solid dosage forms of pregabalin are available. However, solid dosage forms such as tablet or capsule are disadvantageous for pediatric and geriatric patients and people who have swallowing difficulties. Furthermore, they are not preferred by most patients. Therefore, the formulations according to the present invention have been prepared in the form of effervescent formulations which have fast dissolution and impact, long shelf life and which are easy-to-use for patients and convenient for patients who have swallowing difficulties.

As a result of the studies in the scope of the present invention, the inventor has found that use of vitamin B₁₂ as the second active agent in these effervescent formulations comprising pregabalin has provided a higher therapeutic effect than expected in the treatment of epilepsy, central nervous system disorders, Parkinson's disease; Huntington's disease; tardive dyskinesia; spasticity; cerebral ischemia; postherpetic neuralgia; social phobia; fibromyalgia and spinal cord injury induced chronic pains; neuropathic pains associated with diabetic peripheral neuropathy and common anxiety disorder and in treatment and/or prevention of anemia diseases such as macrocytic anemia, megaloblastic anemia and pernicious anemia.

Vitamin B₁₂ or cyanocobalamineis a complex vitamin. Its chemical name is coalpha-(alpha-(5,6- dimethylbenzimidazolyl))- cobeta-cyanocobamide and its chemical structure is illustrated with Formula II.

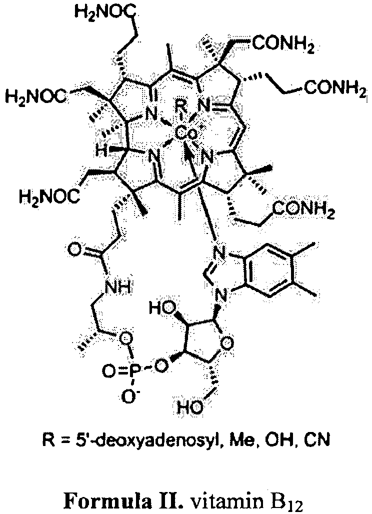

Vitamin B₁₂ is found in fish, shellfish, meat and milk products. It is required for good digestion, nutrition, protein synthesis, carbohydrate and fat metabolism. Vitamin B₁₂ prevents nerve damage, provides fertility in women, cell production and long life, eases normal growth of nerve endings, helps improve memory and learning.

The present invention discloses new, user- friendly, therapeutically advantageous effervescent formulations comprising pregabalin and vitamin B₁₂ in combined form which have fast dissolution and/or dispersion and higher bioavailability as compared to solid dosage forms in the prior art.

In other words, a characteristic feature of the formulations of the present invention is to comprise vitamin B₁₂ together with pregabalin as the active agent.

Another characteristic feature of the formulations of the present invention is that the formulations are in effervescent powder, tablet and granule forms having the advantages of both tablet and suspension forms together, and they remove the problems encountered in those dosage forms. Effervescent dosage forms are beneficial especially for the patients having swallowing difficulties and for paediatrics.

In another aspect, the present invention provides a pharmaceutical formulation in effervescent form comprising pregabalin and vitamin B₁₂ in combined form optionally together with at least one pharmaceutically acceptable excipient.

Another characteristic feature of the formulations of the present invention is that said formulations comprise
1. Pregabalin as a GABA analogue,
2. Vitamin B₁₂,
3. At least one pharmaceutically acceptable effervescent couple and
4. At least one pharmaceutically acceptable excipient.

In another aspect, pregabalin and vitamin B₁₂ comprised in the formulation of the present invention can be in the form of their free bases, pharmaceutically acceptable salts, racemates, solvates, hydrates, anhydrates, different polymorphic forms and amorphous forms, preferably in their free base forms.

The effervescent formulations of the present invention are used after dissolved preferably in a glass of water or in another suitable liquid.

A characteristic feature of the present invention is that total active agent amount comprised in the pharmaceutical formulation is in the range of 0.01 - 60% by weight, preferably in the range of 1 - 20% by weight.

Another characteristic feature of the pharmaceutical formulations of the present invention is to comprise pregabalin in the range of 20 - 500 mg in proportion to total formulation weight.

Another characteristic feature of the pharmaceutical formulations of the present invention is to comprise pregabalin in the range of 100 - 400 mg in proportion to total formulation weight.

Another characteristic feature of the pharmaceutical formulations of the present invention is to comprise pregabalin in the range of 250 - 350 mg in proportion to total formulation weight.

Another characteristic feature of the pharmaceutical formulations of the present invention is to comprise vitamin B₁₂ in the range of 0.01 - 10 mg in proportion to total formulation weight.

Another characteristic feature of the pharmaceutical formulations of the present invention is to comprise vitamin B₁₂ in the range of 0.01 - 5 mg in proportion to total formulation weight.

Another characteristic feature of the pharmaceutical formulations of the present invention is to comprise vitamin B₁₂ in the range of 0.01 - 3 mg in proportion to total formulation weight.

It has been seen that the highest therapeutic effect is obtained in the case that the ratio of pregabalin/vitamin B₁₂ is in the range of 20:1 to 400:1 by weight in the formulations of the present invention.

In other aspect, use of the two active agents in this ratio enable the final effervescent dosage form to reach sufficient dissolution profile and to be produced in a size to provide the ease required in storage and carrying phases.

According to this, another characteristic feature of the effervescent formulations of the present invention is that the ratio of pregabalin/vitamin B₁₂ is in the range of 20:1 to 400:1 by weight.

Another characteristic feature of the effervescent formulations of the present invention is that the ratio of pregabalin/vitamin B₁₂ is more preferably in the range of 100:1 to 350:1 by weight.

The effervescent formulations of the present invention can be prepared in the form of effervescent powder, tablet and granule.

The term "effervescent powder, tablet and granule" refers to effervescent tablets, effervescent granules, effervescent powders, effervescent tablets. The preferred dosage form according to the present invention is effervescent tablet.

The effervescent formulations of the present invention can comprise pregabalin and vitamin B₁₂ in a single dosage form while they also can comprise the active agents in different dosage forms. The preferred formulations according to the present invention comprise the two active agents in a single dosage form.

The effervescent formulations of the present invention can comprise at least one pharmaceutically acceptable excipient selected from a group comprising flavouring agent, solvent or solvent mixtures, binder, lubricant, sweetener and taste regulating agent or combinations thereof along with pregabalin and vitamin B₁₂ as the active agent and at least one effervescent couple.

The characteristic feature of the effervescent formulations of the present invention is that said formulations comprise at least one pharmaceutically acceptable effervescent couple in the range of 75 - 95% by weight.

The term "effervescent couple" refers to to the combination comprising at least one effervescent acid and at least one effervescent base.

Another characteristic feature of the effervescent formulations of the present invention is that total formulation comprises the effervescent acid in the range of 30 - 70% by weight, preferably in the range of 40 - 55% by weight, and the effervescent base is in the range of 20-60% by weight, preferably in the range of 30-45% by weight.

The pharmaceutically acceptable effervescent acids that can be used in the effervescent formulations of the present invention are selected from a group comprising citric acid and acetic acid, tartaric acid, fumaric acid, adipic acid, malic acid or their pharmaceutically acceptable hydrates, anhydrates or combinations thereof.

The pharmaceutically acceptable effervescent bases that can be used in the effervescent formulations of the present invention are selected from a group comprising potassium carbonate, potassium bicarbonate, potassium citrate, potassium hydroxide, sodium carbonate and sodium hydrogen carbonate or combinations thereof.

The pharmaceutically acceptable taste regulating agent of the present invention can be selected from a group comprising sodium chloride, potassium chloride or combinations thereof.

Preferably, the present invention comprises saccharose, aspartame, sodium chloride or a mixture comprising a combination thereof as the taste regulating agent.

The pharmaceutically acceptable sweetener of the present invention can be selected from a group comprising acesulfame potassium, acesulfame, aspartame, fructose, dextrose, glucose, lactitol, maltitol, xylitol, sorbitol, maltose, saccharine, saccharine sodium, sodium cyclamate, sucralose, sucrose or combinations thereof.

The pharmaceutically acceptable binder of the present invention can be selected from a group comprising ethyl cellulose, hydroxyethyl cellulose, methyl cellulose, hydroxymethyl cellulose, hydroxypropyl cellulose, sorbitol, gelatine, hypromellose, magnesium aluminium silicate, maltodextrin, polyethylene oxide, polyvinylpyrrolidone, povidone and water or combinations thereof.

The pharmaceutically acceptable lubricant of the present invention can be selected from a group comprising magnesium stearate, polyethylene glycol 4000 (Peg 4000), polyethylene glycol 6000 (Peg 6000), sodium lauryl sulphate, starch, talc or combinations thereof.

The pharmaceutically acceptable flavouring agent of the present invention can be selected from a group comprising sour cherry flavour, grapefruit flavour, lemon flavour, grape flavour, pear flavour, orange flavour, apricot flavour or combinations thereof.

The effervescent formulation of the present invention comprises
- Pregabalin in the range of 0.5-20% by weight,
- Vitamin B₁₂ in the range of 0.01-3% by weight,
- Effervescent acid in the range of 40 - 55% by weight,
- Effervescent base in the range of 30 - 45% by weight and
- At least one other pharmaceutically acceptable excipient in the range of 0.1 - 10% by weight.

The formulations of the present invention can be produced by any method existing in the prior art. Dry granulation, dry blending, direct compression, wet granulation can be listed among these methods.

The effervescent formulations of the present invention can be produced according to any methods given below:
1. Mixing pregabalin and vitamin B₁₂ as the active agent homogeneously and adding at least one of the excipients given above if required; optionally treating the mixture with at least one pharmaceutically acceptable lubricant; compressing this mixture in tablet form under a tablet compression force according to the present invention,
2. Optionally wet-granulating the mixture obtained by mixing pregabalin and vitamin B₁₂ homogeneously as the active agent and adding at least one of the excipients given above with the granulation solution comprising at least one excipient if required; drying the obtained granules; optionally treating the granules with at least one pharmaceutically acceptable lubricant and compressing the granules in tablet form under a tablet compression force according to the present invention,
3. Optionally wet-granulating at least one of the excipients given above with the granulation solution comprising at least one excipient; drying the granules obtained; adding pregabalin and vitamin B₁₂ and optionally at least one excipient into the dry granules and mixing them; treating the granules optionally with at least one pharmaceutically acceptable lubricant and compressing them in tablet form under a tablet compression force according to the present invention,
4. Dry-granulating the mixture obtained by mixing pregabalin and vitamin B₁₂ homogeneously as the active agent and adding at least one of the excipients given above if required and compressing the obtained granules in tablet form under a tablet compression force according to the present invention,
5. Preparing the granulation solution with vitamin B₁₂ as the active agent and at least one pharmaceutical excipient; granulating other pharmaceutically acceptable excipients, effervescent couple and pregabalin with the granulation solution; compressing the obtained granules in tablet form under a tablet compression force according to the present invention,
6. In the case that two active agents are used, the formulations can be produced by a method composed of using any abovementioned methods separately for the active agent formulations and combining the formulations obtained afterwards.

In another aspect, the formulation of the present invention can be used in prevention of the seizures, in reducing the number of the seizures or in the treatment of the patients diagnosed with epilepsy.

In another aspect, the formulation of the present invention can be prepared as a drug formulation which is effective in the treatment of epilepsy, central nervous system disorders, Parkinson's disease; Huntington's disease; tardive dyskinesia; spasticity; cerebral ischemia; postherpetic neuralgia; social phobia; fibromyalgia and spinal cord injury induced chronic pains; neuropathic pains associated with diabetic peripheral neuropathy and common anxiety disorder and in treatment and/or prevention anemia diseases such as macrocytic anemia, megaloblastic anemia and pernicious anemia.

The pharmaceutical formulation of the present invention and its preparation methods can be explained with the examples below.

### Example 1:

Effervescent dosage form comprising pregabalin and vitamin B₁₂ in combined form;

| **Component** | **Amount (%)** |
|---|---|
| **Active agent** | |
| Pregabalin | 13.0 |
| Vitamin B₁₂ | 0.5 |

| **Excipients** | |
|---|---|
| Taste regulating agent | 2.5 |
| Effervescent acid | 44.0 |
| Effervescent base | 34.0 |
| Lubricant | 1.0 |
| Binder | 3.0 |
| Sweetener | 1.0 |
| Flavouring agent | 1.0 |
| **Total** | 100.0 |

The formulation comprising pregabalin, vitamin B₁₂ and the basic agent is granulated with the granulation solution comprising a pharmaceutically acceptable binder. The granulation solution used herein can comprise a solvent in addition to the binder. The granules are dried and sieved. The effervecent acid, the taste regulating agent and the binder are mixed and added into this formulation, they are granulated, and the granules are dried. The sweetener, the flavouring agent and the lubricant are added and the formulation is finalised. Optionally tablet compression is performed.

### Example 2:

Effervescent dosage form comprising pregabalin and vitamin B₁₂ in combined form;

| **Content** | **Amount (%)** |
|---|---|
| **Active agent** | |
| Pregabalin | 1.0 |
| Vitamin B₁₂ | 0.5 |

| **Excipients** | |
|---|---|
| Taste regulating agent | 2.5 |
| Effervescent acid | 50.5 |
| Effervescent base | 39.5 |
| Lubricant | 1.0 |
| Binder | 3.0 |
| Sweetener | 1.0 |
| Flavouring agent | 1.0 |
| **Total** | 100.0 |

The granulation solution is prepared with vitamin B₁₂ and a pharmaceutically acceptable binder. The granulation solution used herein can comprise a solvent in addition to the binder. The effervescent couple, the taste regulating agent and the binder are mixed. The obtained formulation is granulated with the granulation solution. The granules are dried. The sweetener and pregabalin are added into this formulation. The flavouring agent and the lubricant are added and the formulation is finalised. Optionally, tablet compression is performed.

## Claims

1. A pharmaceutical formulation comprising pregabalin and vitamin B₁₂, **characterized in that** said formulation is in effervescent form.

2. The effervescent formulation according to claim 1, **characterized in that** the amount of the active agent comprised in the pharmaceutical formulation is in the range of 0.01-60% by weight, preferably in the range of 1- 20% by weight, in the total formulation.

3. The effervescent formulation according to claims 1-2, **characterized in that** said formulation comprises pregabalin in the range of 20 - 500 mg, preferably in the range of 100 - 400 mg, more preferably in the range of 250 - 350 mg, per unit dosage form.

4. The effervescent formulation according to claims 1-3, **characterized in that** said formulation comprise vitamin B₁₂ in the range of 0.01 - 10 mg, preferably in the range of 0.01 - 5 mg, more preferably in the range of 0.01 - 3, per unit dosage form.

5. The effervescent formulation according to any preceding claim, **characterized in that** the ratio of the active agents composing the effervescent tablet is in the range of 20:1 to 400:1 by weight, preferably in the range of 100:1 to 350:1 by weight.

6. The effervescent formulation according to claim 1, **characterized in that** said formulation comprises at least one pharmaceutically acceptable effervescent couple comprising at least one effervescent acid and at least one effervescent base, preferably wherein said formulation comprises at least one effervescent couple in the range of 75 - 95% by weight.

7. The effervescent formulation according to claim 6, **characterized in that** the effervescent acid used in said formulation is selected from a group comprising citric acid, acetic acid, tartaric acid, fumaric acid, adipic acid, malic acid or their pharmaceutically acceptable hydrates, anhydrates or combinations thereof.

8. The effervescent formulation according to claim 7, **characterized in that** said formulation comprises the effervescent acid in the range of 30 - 70% by weight, preferably in the range of 40-55% by weight.

9. The effervescent formulation according to claim 6 **characterized in that** the effervescent base used in said formulation is selected from a group comprising potassium carbonate, potassium bicarbonate, potassium citrate, potassium hydroxide, sodium carbonate and sodium hydrogen carbonate or combinations thereof.

10. The effervescent formulation according to claim 9, **characterized in that** said formulation comprises the effervescent base in the range of 20-60% by weight, preferably in the range of 30-45% by weight.

11. The effervescent formulation according to any preceding claim, **characterized in that** said formulation comprises at least one pharmaceutically acceptable excipient, wherein the at least one excipient is preferably selected from a group comprising flavouring agent, solvent or solvent mixtures, binder, lubricant, sweetener and taste regulating agent or combinations thereof.

12. The effervescent formulation according to any preceding claims, **characterized in that** said formulation is in the form of effervescent tablets, effervescent granules, effervescent powders.

13. The effervescent formulation according to any preceding claim, **characterized in that** said formulations arc produced by one of the dry granulation, dry blending, direct compression, wet granulation methods.

14. The effervescent formulation according to any preceding claim for use in the treatment of epilepsy, central nervous system disorders, Parkinson's disease; Huntington's disease; tardive dyskinesia; spasticity; cerebral ischemia; postherpetic neuralgia; social phobia; fibromyalgia and spinal cord injury induced chronic pains; neuropathic pains associated with diabetic peripheral neuropathy and common anxicty disorder and anemia diseases such as macrocytic anemia, megaloblastic anemia and pernicious anemia.

## Patentansprüche

1. Pharmazeutische Formulierung, die Pregabalin und Vitamin B₁₂ enthält, **dadurch gekennzeichnet, dass** die genannte Formulierung in sprudelnder Form vorliegt.

2. Sprudelnde Formulierung nach Anspruch 1, **dadurch gekennzeichnet, dass** die in der pharmazeutischen Formulierung enthaltene Menge des Wirkstoffs im Bereich von 0.01-60 Gew.-%, vorzugsweise im Bereich von 1 -20 Gew.-%, in der Gesamtformulierung liegt.

3. Sprudelnde Formulierung nach den Ansprüchen 1 bis 2, **dadurch gekennzeichnet, dass** die genannte Formulierung Pregabalin im Bereich von 20 - 500 mg, vorzugsweise im Bereich von 100 - 400 mg, noch bevorzugter im Bereich von 250 - 350 mg, pro Einheitsdosierungsform enthält.

4. Sprudelnde Formulierung nach den Ansprüchen 1 bis 3, **dadurch gekennzeichnet, dass** die genannte Formulierung Vitamin B₁₂ im Bereich von 0.01 - 10 mg, vorzugsweise im Bereich von 0.01 - 5 mg, noch bevorzugter im Bereich von 0.01 - 3, pro Einheitsdosierungsform enthält.

5. Sprudelnde Formulierung nach einem der vorhergehenden Ansprüche, **dadurch gekennzeichnet, dass** das Verhältnis der Wirkstoffe, aus denen die Sprudeltablette besteht, im Bereich von 20:1-400:1, vorzugsweise im Bereich von 100:1-350:1, liegt, nach Gewicht.

6. Sprudelnde Formulierung nach Anspruch 1, **dadurch gekennzeichnet, dass** die Formulierung mindestens ein pharmazeutisch verträgliches Sprudelpaar enthält, das mindestens eine Sprudelsäure und mindestens eine Sprudelbase enthält, wobei die genannte Formulierung vorzugsweise mindestens ein Sprudelpaar im Bereich von 75 - 95 Gew.-% enthält.

7. Sprudelnde Formulierung nach Anspruch 6, **dadurch gekennzeichnet, dass** die in dieser Formulierung verwendete Sprudelsäure aus einer Gruppe ausgewählt ist, die Zitronensäure, Essigsäure, Weinsäure, Fumarsäure, Adipinsäure, Apfelsäure oder deren pharmazeutisch verträgliche Hydrate, Anhydrate oder Kombinationen davon enthält.

8. Sprudelnde Formulierung nach Anspruch 7, **dadurch gekennzeichnet, dass** die genannte Formulierung die Sprudelsäure im Bereich von 30 - 70 Gew.-%, vorzugsweise im Bereich von 40-55 Gew.-%, enthält.

9. Sprudelnde Formulierung nach Anspruch 6, **dadurch gekennzeichnet, dass** die in der Formulierung verwendete Sprudelbase aus einer Gruppe ausgewählt ist, die Kaliumcarbonat, Kaliumbicarbonat, Kaliumcitrat, Kaliumhydroxid, Natriumcarbonat und Natriumhydrogencarbonat oder Kombinationen davon umfasst.

10. Sprudelnde Formulierung nach Anspruch 9, **dadurch gekennzeichnet, dass** die genannte Formulierung die Sprudelbase im Bereich von 20-60 Gew.-%, vorzugsweise im Bereich von 30-45 Gew.-%, enthält.

11. Sprudelnde Formulierung nach einem der vorhergehenden Ansprüche, **dadurch gekennzeichnet, dass** die Formulierung mindestens einen pharmazeutisch verträglichen Hilfsstoff enthält, wobei der mindestens eine Hilfsstoff vorzugsweise aus einer Gruppe ausgewählt ist, die einen Aromastoff, ein Lösungsmittel oder Lösungsmittelgemische, ein Bindemittel, ein Gleitmittel, ein Süßungsmittel und ein geschmacksregulierendes Mittel oder Kombinationen davon enthält

12. Sprudelnde Formulierung nach einem der vorhergehenden Ansprüche, **dadurch gekennzeichnet, dass** die genannte Formulierung in Form von Sprudeltabletten, Sprudelgranulat, Sprudelpulver ist.

13. Sprudelnde Formulierung nach einem der vorhergehenden Ansprüche, **dadurch gekennzeichnet, dass** die genannten Formulierungen durch eines der Verfahren Trockengranulation, Trockenmischung, Direktverdichtung, Nassgranulation hergestellt werden.

14. Sprudelnde Formulierung nach einem der vorhergehenden Ansprüche zur Verwendung bei der Behandlung von Epilepsie, Störungen des Zentralnervensystems, Parkinson-Krankheit, Huntington-Krankheit, tardiver Dyskinesie, Spastizität, zerebraler Ischämie, postherpetischer Neuralgie; Sozialphobie; Fibromyalgie und Rückenmarkverletzungen induzierte chronische Schmerzen; neuropathische Schmerzen in Verbindung mit diabetischer peripherer Neuropathie und häufigen Angststörungen und Anämiekrankheiten wie makrozytäre Anämie, megaloblastische Anämie und perniziöse Anämie.

## Revendications

1. Formulation pharmaceutique comprenant de prégabaline et de vitamine B₁₂, **caractérisée en ce que** ladite formulation est sous forme effervescente.

2. Formulation effervescente selon la revendication 1, **caractérisée en ce que** la quantité de l'agent actif contenue dans la formulation pharmaceutique est comprise entre 0.01-60% en poids, de préférence entre 1-20% en poids, dans la formulation totale.

3. Formulation effervescente selon la revendication 1-2, **caractérisée en ce que** ladite formulation comprend de prégabaline dans la gamme de 20 - 500 mg, de préférence dans la gamme de 100 - 400 mg, plus préférablement dans la gamme de 250 - 350 mg, par forme de dosage unitaire.

4. Formulation effervescente selon la revendication 1-3, **caractérisée en ce que** ladite formulation comprend de vitamine B₁₂ dans la gamme de 0.01 - 10 mg, de préférence dans la gamme de 0.01 - 5 mg, plus préférablement dans la gamme de 0.01 - 3, par forme de dosage unitaire.

5. Formulation effervescente selon l'une quelconque des revendications précédentes, **caractérisée en ce que** le rapport des agents actifs composant le comprimé effervescent est compris entre 20:1 à 400:1 en poids, de préférence entre 100:1 à 350:1 en poids.

6. Formulation effervescente selon la revendication 1, **caractérisée en ce que** ladite formulation comprend au moins un couple effervescent pharmaceutiquement acceptable comprenant au moins un acide effervescent et au moins une base effervescente, de préférence dans laquelle ladite formulation comprend au moins un couple effervescent dans la gamme de 75 - 95% en poids.

7. Formulation effervescente selon la revendication 6, **caractérisée en ce que** l'acide effervescent utilisé dans ladite formulation est choisi dans un groupe comprenant l'acide citrique, l'acide acétique, l'acide tartrique, l'acide fumarique, l'acide adipique, l'acide malique ou leurs hydrates, anhydrates ou combinaisons pharmaceutiquement acceptables.

8. Formulation effervescente selon la revendication 7, **caractérisée en ce que** ladite formulation comprend l'acide effervescent dans la gamme de 30 - 70% en poids, de préférence dans la gamme de 40 - 55% en poids.

9. Formulation effervescente selon la revendication 6 **caractérisée en ce que** la base effervescente utilisée dans ladite formulation est choisie dans un groupe comprenant le carbonate de potassium, le bicarbonate de potassium, le citrate de potassium, l'hydroxyde de potassium, le carbonate de sodium et l'hydrogénocarbonate de sodium ou leurs combinaisons.

10. Formulation effervescente selon la revendication 9, **caractérisée en ce que** ladite formulation comprend la base effervescente dans la gamme de 20-60% en poids, de préférence dans la gamme de 30-45% en poids.

11. Formulation effervescente selon l'une quelconque des revendications précédentes, **caractérisée en ce que** ladite formulation comprend au moins un excipient pharmaceutiquement acceptable, dans laquelle le au moins un excipient est de préférence choisi dans un groupe comprenant un agent aromatisant, un solvant ou des mélanges de solvants, un liant, un lubrifiant, un édulcorant et un agent de régulation du goût ou des combinaisons de ceux-ci.

12. Formulation effervescente selon l'une quelconque des revendications précédentes, **caractérisée en ce que** ladite formulation se présente sous forme de comprimés effervescents, de granulés effervescents, de poudres effervescentes.

13. Formulation effervescente selon l'une quelconque des revendications précédentes, **caractérisée en ce que** lesdites formulations sont produites par l'une des méthodes suivantes : granulation sèche, mélange à sec, compression directe, granulation humide.

14. Formulation effervescente selon l'une quelconque des revendications précédentes pour le traitement de l'épilepsie, des troubles du système nerveux central, de la maladie de Parkinson, de la maladie de Huntington, de la dyskinésie tardive, de la spasticité, de l'ischémie cérébrale, de la névralgie post-zostérienne; de la phobie sociale ; fibromyalgie et lésions de la moelle épinière provoquant des douleurs chroniques ; douleurs neuropathiques associées à une neuropathie périphérique diabétique et un trouble anxieux courant et maladies anémiques telles que l'anémie macrocytaire, l'anémie mégaloblastique et l'anémie pernicieuse.
